# EUROPEAN PATENT SPECIFICATION

(11) **EP 1 057 491 B1**
(45) Date of publication and mention of the grant of the patent: **19.06.2013**
(21) Application number: 99905256.6
(22) Date of filing: 19.02.1999
(51) Int. Cl.: A61K 38/04, A61K 38/06, C07K 14/36, C07K 5/093, C12P 1/06, A61K 38/03, A61K 45/06

(54) **Preventives/remedies for skin aging**
Vorbeuge- und Heilmittel gegen die Hautalterung
Moyens preventifs/remèdes contre le vieillissement de la peau

(30) Priority: 24.02.1998 JP 4147998
(43) Date of publication of application: 06.12.2000
(73) Proprietor: Astellas Pharma Inc., Chuo-ku, Tokyo (JP)
(72) Inventor: YABUTA, Tsuguo, Ibaraki-shi,Osaka 567-0862 (JP); YASUMURA, Mitsuru, Nishinomiya-shi, Hyogo 662-0053 (JP); NAKAHARA, Kunio, Kawabe-gun, Hyogo 666-0261 (JP); FURUKAWA, Yusuke, Nara-shi, Nara 631-0845 (JP); NOMURA, Kazuhiko, Takatsuki-shi, Osaka 569-0815 (JP); MURAKAMI, Manabu, Suita-shi, Osaka 565-0806 (JP)
(74) Representative: Gille Hrabal
(86) International application number: PCT/JP1999/000761
(87) International publication number: WO 1999/043352

(56) References cited:
- EP-A- 0 387 712
- EP-A- 0 519 354
- EP-A1- 0 465 895
- EP-A1- 0 585 155
- EP-A2- 0 494 071
- WO-A-96/28008
- JP-A- 4 279 600
- JP-A- 4 297 446
- JP-A- 6 184 192
- US-A- 5 614 489
- GODEAU G ET AL: "Morphometric analysis of the degradation of human skin elastic fibres by human leukocyte elastase (EC 3-4-21-37) and human skin fibroblast elastase (EC 3-4-24)", PATHOLOGIE ET BIOLOGIE, L'EXPANSION SCIENTIFIQUE FRANCAISE, PARIS, FR, vol. 36, no. 9, 1 November 1988 (1988-11-01), pages 1133-1138, XP008158417, ISSN: 0369-8114

## Description

### TECHNICAL FIELD

This invention relates to a cosmetic and non-therapeutic method for the prophylaxis and treatment of dermal aging wherein a substance having a human leukocyte elastase inhibitory activity, which is 3(RS)-[[4-(carboxymethylaminocarbonyl)phenylcarbonyl]-L-valyl-L-prolyl]amino- ,1,1-trifluoro-4-methyl-2-oxopentane or its sodium salt, is used topically.

### BACKGROUND ART

Aging of the skin is said to begin after the third decade of life. Obvious signs of aging include decreases in the moistness, gloss, smoothness, tonus, etc. of the skin and an increased number of wrinkles. These are suspected to result from the morphological and functional changes of the organs and tissues making up the skin. Thus, aging is accompanied by thinning of the epidermis and loss of oxytalan fiber in the papillary layer of the dermis.

Dermal aging, epitomized by wrinkling of the skin, is a serious beauty problem for women but no satisfactory remedy has been available to this day.

JP 6-184 192 corresponding to EP 0 585 155 discloses the use of human Leukocyte Elastase inhibitors In the treatment of skin ageing, which, however, have a different chemical structure than FK706.

WO 96/28008 discloses a composition comprising a combination of an elastase inhibitor with a compound that affects formation of Amadori products for controlling skin ageing and/or increasing skin elasticity.

EP 0 494 071 discloses trifluoromethylketone tripeptide derivatives, Inter alia FK706, and reports their human leukocyte elastase inhibiting activity. Treatment of dermal ageing or wrinkles is not addressed In this document.

US 5,614,489 discloses a cosmetic composition comprising a vehicle for topical administration and a collagenase and/or elastase inhibitor, to reduce and substantially prevent the appearance of fine lines and wrinkles. The inhibitors are structurally different from FK706.

EP 0 465 895 discloses WS7622A mono- or disulfate, and its human leukocyte ealstase inhibitory activity.

Godeau G. et al, Pathologie at Biologie, L'expansion scientifique française, Paris, vol. 36, no.9, 1. November 1988, p. 1133-1138 discloses a study of degradation of human skin preelastic fibres and that of mature skin elastic fibres by human leukocyte elastase and purified human fibroblast elastase by morphometric analysis.

The present inventors discovered that a substance having human leukocyte elastase inhibitory activity Is effective in the prevention and treatment of dermal aging and have perfected this invention.

### DISCLOSURE OF INVENTION

This invention is directed to a cosmetic and non-therapeutic method for the prophylaxis and treatment of dermal aging wherein a substance having a human leukocyte elastase inhibitory activity, which is 3(RS)-[[4-(carboxymethylaminocarbonyl)phenylcarbonyl]-L-valyl-L-prolyl]amino-1,1,1-trifluoro-4-methyl-2-oxopentane or its sodium salt, is used topically

The substance having human leukocyte elastase inhibitory activity which is used as the active ingredient in the method for prophylaxis and treatment of dermal aging is substance FK706 defined below.

As a reference compound there is mentioned (not according to the invention) WS7622A mono- or disulfate and pharmaceutically acceptable salts thereof; among these, WS7622A disulfate ester disodium salt and WS7622A disulfate ester dipotassium salt are known substances which have the physicochemical properties described in JP Kokal H4-279600 [EP 0 465 895 A1].

WS7622A disulfate ester disodium salt Is hereinafter sometimes referred to briefly as FR134043 (not according to the invention; used for reference).

The above-mentioned Substance WS7622A can be produced by growing Streptomyces resistomycificus No. 7622, which strain has been deposited with National Institute of Bioscience and Human Technology, one of the international culture collections under Budapest Treaty, with the accession number of FERM BP-2306 assigned.

The HLE inhibitor of the present invention is 3 (RS)-[[4-(carboxymethylaminocarbonyl)phenyl-carbonyl]-L-valyl-L-prolyl]amino-1,1,1-trifluoro-4-methyl-2-oxopentane or its sodium salt (this sodium salt will sometimes be referred to briefly as FK706) as defined In claim 1.

The compound mentioned in the above paragraph is a known compound as described in JP Kokal H4-297446 [EP 0 494 071 A2], for instance.

For the purposes of this invention, the substance having human leukocyte elastase inhibitory activity as defined in claim 1 is efficacious and can be used in a cosmetic and non-therapeutic method for the prevention and treatment of dermal aging in general. More particularly, it can be indicated for the prevention and treatment of decreases in moistfulness, sheen, smoothness, and tonus of the skin and even Increased wrinkling, or the prevention and treatment of skin flaccidity. These signs of dermal aging are suspected to arise from morphological and functional changes of the organs and tissues making up the skin, and actually, thinning of the horny layer of the epidermis and loss of oxytalan fiber in the papillary layer of the corium are noted.

The signs of dermal aging being as mentioned above, the efficacy of the substance having human leukocyte elastase inhibitory activity as defined in claim 1 is particularly pronounced for the elimination or diminution of wrinkles or prevention of increased wrinkling, improvement of skin texture (fineness, handle) and amelioration of the shade of the skin (shadowy complexion).

Furthermore, said substance is efficacious for promoting neogenesis of oxytalan fiber In the region of dermal papillae and neogenesis of collagen fibrils in the dermis immediately beneath the epidermis, and for increasing the thickness of the epidermis, among others.

The following is an example of experimentation relevance to this invention.

### Object of experiment:

The therapeutic efficacy of neutrophil (leukocyte) elastase inhibitors In "dermal aging" was evaluated in hairless dogs.

### Experimental animals:

Two adult (old) experimental hairless dogs constructed by cross-breeding of a Mexican hairless dog and a beagle dog were used.

Old dogs presenting with age-associated fine "wrinkles", not observed in young dogs, on the body surface were used as subject animals.
No. 8807 (10 yr old, male)
No. 8808 (10 yr old, female)

### Investigational drugs:

1) FK706.0.2%
2) FK706.0.02%
3) FR134043.0.2% (Reference)
4) FR134043.0.02% (Reference)
5) Polyethylene glycol (PEG) (solvent control)
(PEG was used as the solvent for drugs 1 - 4)

### Administration:

Each drug was applied in one location (a total of 5 locations) on the back (5 x 5 cm) of each dog. The drug was applied in a dosing volume of about 4 µL/cm² once dally (except on Saturdays, Sundays and holidays) for 3 months.

### Evaluation items:

1) Skin condition: Gross observation and observation with a video macroscope
2) Histology: Observation of histological changes in biopsy samples by HE (hematoxylin-eosin) stain (general staining), van Gieson's stain (staining of collagen fiber) and Weigert's stain (staining of elastin fiber)
3) Skin thickness: Using H-E stained tissue samples, changes in thickness of the epidermis (excluding the horny layer) were studied (only the location treated with FK706 0.2%, which showed a marked improvement in skin condition, was evaluated)

### Results:

### 1) Skin condition (remission of wrinkles)

### (Gross observation)

No. 8807: In the elimination or remission of wrinkles, the order of efficacy was FK706.0.1 % > FK706.0.02% > FR134043.0.2% = FR134043.0.02%. This finding of remission was accompanied by improvements in skin texture (fineness, handle) and paralleled depigmentation of the skin (change to fair - rosy skin).
No. 8808: The order of efficacy was FK706.0.2% > FK706.0.02% > FR134043.0.2% = FR134043.0.02%.

In neither dog was found a side effect.

### (Observation with a video macroscope)

The findings were substantially identical to the results of gross observation.
No. 8807: The order of efficacy was FK706.0.2% > FK706.0.02% > FR134043.0.2% > FR134043.0.02%
No. 8808: The order of efficacy was FK706.0.2% = FK706.0.02% > FR134043.0.2% = FR134043.0.02%.

### 2) Histological findings

van Gleson's stain:
   Neogenesis of a thin layer of collagen fiber was found across the boundary between the epidermis and dermis in the case of FK706.0.2% (Dog No. 8807)
Weigert's strain:
   Growth of elastin fibrils (suspected to be oxytalan fiber which is said to disappear as increasing age) was found across the boundary between the epidermis and dermis (Dog No. 8807).

### 3) Evaluation of skin thickness

The thickness of the epidermis was increased significantly (p<0.05 in both Dog 8807 and Dog 8808).
No. 8807: 17.0+/-1.9 µm → 26.8±5.9 µm (mean+/-SD)
No. 8808: 24,6+/-3.6 µm → 42.0±9.0 µm

The compound used in the claimed method for prophylaxis and treatment of dermal aging as provided by this Invention is usually applied in the form of a preparation for external application (e.g. lotion, ointment, patch, liniment, aerosol, suspension, emulsion) or an external powder (e.g. an enzyme facial cleanser. Furthermore, it can be used in such formulations as facial cleansers, facial wash-off/emolient preparations, and bath preparations. Where necessary, a diluent or disintegrator (e.g. sucrose, lactose, starch, crystalline cellulose, low-substitutlon-degree hydroxypropylcellulose, synthetic aluminum silicate), a binder (e.g. cellulose, methylcellulose, hydroxypropylcellulose, hydroxypropylmethylcellulose, polypropylpyrrolidone, polyvinylpyrrolidone, gelatin, gum arabic, polyethylene glycol), a coloring agent, a sweetener and a lubricant (e.g. magnesium stearate), among others, can be additionally dispersed in such formulations.

The level of use of the compound in the claimed method for prophylaxis and treatment of dermal aging according to this invention depends on the symptoms and other factors but, generally speaking, the recommended dose of an external dosage form, in terms of the concentration of the substance having human leukocyte elastase inhibitory activity orits sodium salt as defined in claim 1, can be judiciously selected from the range of 0.001 - 20%, preferably about 0,01 - 10%.

## Claims

1. Cosmetic and non-therapeutic method for the prophylaxis and treatment of dermal aging wherein a substance having a human leukocyte elastase inhibitory activity, which Is 3(RS)-[[4-(carboxymethylaminocarbonyl)phenylcarbonyl]-L-volyl-L-prolyl]amino-1,1,1-trifluoro-4-methyl-2-oxopentane or Its sodium salt, is used topically.

2. Cosmetic method as claimed in claim 1 for the prophylaxis and treatment of wrinkles,

## Patentansprüche

1. Kosmetisches und nicht-therapeutisches Verfahren zur Prophylaxe und Behandlung der Hautalterung, worin eine Substanz mit humaner Leukozyten-Elastase Inhibitoraktivität, die 3(RS)-[[4-(carboxymethylaminocarbonyl)phenylcarbonyl]-L-valyl-L-prolyl]amino-1,1,1-trifluoro-4-methyl-2-oxopentan oder dessen Natriumsalz ist, topisch angewendet wird.

2. Kosmetisches Verfahren nach Anspruch 1 zur Prophylaxe und Behandlung von Falten.

## Revendications

1. Procédé cosmétique et non thérapeutique pour la prophylaxie et le traitement du vieillissement dermique, dans lequel une substance ayant une activité inhibitrice vis-à-vis de l'élostase leucocytaire humaine, qui est 3(RS)-[[4-(carboxyméthylaminocarbonyl)phénylcarbonyl]-L-valyl-L-prolyl]amino-1,1,1,-trifluoro-4-méthyl-2-oxopentane ou son sel de sodium, est utilisée topiquement.

2. Procédé cosmétique selon la revendication 1 pour la prophylaxie et le traitement des rides.
